# EUROPÄISCHE PATENTANMELDUNG

(11) **EP 3 081 601 A1**
(43) Veröffentlichungstag der Anmeldung: **19.10.2016**
(21) Anmeldenummer: 15001083.3
(22) Anmeldetag: 15.04.2015
(51) Int. Cl.: C09C 1/00

(54) **PERLGLANZPIGMENTE AUF DER BASIS VON MONOLITHISCH AUFGEBAUTEN SUBSTRATEN**

(71) Anmelder: Schlenk Metallic Pigments GmbH, 91154 Roth (DE)
(72) Erfinder: Huber, Adalbert, 64625 Bensheim (DE); Piech, Fabian, 90530 Wendelstein (DE); Shimizu, Kaiman, 91154 Roth (DE)
(74) Vertreter: Müller-Boré & Partner Patentanwälte PartG mbB

(57) **Zusammenfassung**

Die vorliegende Erfindung betrifft Perlglanzpigmente, Verfahren zu deren Herstellung sowie die Verwendung solcher Perlglanzpigmente, wobei die Perlglanzpigmente monolithisch aufgebaute Substratplättchen aus einem Metalloxid mit einer durchschnittlichen Dicke von 1 bis 40 nm und einem Formfaktor, ausgedrückt durch das Verhältnis der mittleren Größe zur durchschnittlichen Dicke, von mindestens 80, die von mindestens einer im Wesentlichen transparenten Beschichtung A aus mindestens einem niedrigbrechenden Metalloxid und/oder Metalloxidhydrat, das einen Brechungsindex von weniger als 1,8 aufweist, umhüllt sind, und mindestens eine Interferenzschicht in Form einer Beschichtung B aus mindestens einem hochbrechenden Metalloxid, das einen Brechungsindex von mindestens 1,8 aufweist, umfassen.

## Beschreibung

Die vorliegende Erfindung betrifft Perlglanzpigmente, Verfahren zu deren Herstellung sowie die Verwendung solcher Perlglanzpigmente.

Glanzpigmente, insbesondere metallische Glanzpigmente bzw. Metalleffektpigmente finden breite Anwendung in vielen Bereichen der Technik. Sie werden beispielsweise zur Einfärbung von Lacken, Druckfarben, Tinten, Kunststoffen, Gläsern, keramischen Produkten und Zubereitungen der dekorativen Kosmetik eingesetzt. Von besonderer wirtschaftlicher Bedeutung ist die Verwendung von metallischen Glanzpigmenten in Automobillackierungen. Aufgrund ihrer nicht kopierbaren optischen Effekte werden sie auch in der Herstellung fälschungssicherer Wertschriften und Dokumente wie Geldscheine, Schecks, Bank- und Kreditkarten, Eintrittskarten und Tickets, eingesetzt. Sie zeichnen sich vor allem durch ihren reizvollen winkelabhängigen Farbeindruck (Goniochromie) und ihren metallartig wirkenden Glanz aus.

Bei gewöhnlichen Pigmenten entsteht ein Farbeindruck lediglich durch Absorption bestimmter Wellenlängen einfallenden Lichts und Streureflexion. Gängige metallische Effektpigmente reflektieren das einfallende Licht in hohem Maße und erzeugen einen Hell-Dunkel-Flop, aber keinen Farbeindruck. Bei speziellen Perlglanzpigmenten entsteht jedoch aufgrund optischer Interferenzeffekte ein Farbeindruck. Derartige Perlglanzpigmente, die in der Regel auf mindestens einfach beschichteten plättchenförmigen Substraten beruhen, zeigen Interferenzeffekte durch Überlagerung von verschieden gebrochenen und reflektierten Lichtstrahlen. Auf die ebene Oberfläche der beschichteten Substrate einfallendes weißes Licht wird zum Teil an der äußeren Oberfläche der Beschichtung reflektiert. Der andere Teil wird gebrochen und an Grenzflächen, beispielsweise zwischen Beschichtung und Substratoberfläche, reflektiert und erneut gebrochen. Daher kommt es zur Überlagerung von Lichtstrahlen unterschiedlicher Phase. Durch Interferenz des reflektierten Lichts entsteht ein Farbeindruck. Aufgrund der Abhängigkeit der Phasendifferenz vom Einfalls-/Beobachtungswinkei ist auch der Farbeindruck winkelabhängig. Dieser Effekt des Farbwechsels zwischen verschiedenen Reflexionswinkeln wird als Farbflop bezeichnet. Die Phasendifferenz wird u.a. durch die Dicke der Beschichtung(en) beeinflusst, wodurch der entstehende Farbeindruck über die Beschichtungsdicke eingestellt werden kann.

Zu den am längsten bekannten Interferenzpigmenten, die auch Perlglanzpigmente genannt werden, zählt natürliches Fischsilber, welches jedoch aufgrund der Gewinnung und der damit verbundenen hohen Kosten eine untergeordnete Rolle spielt. Neben bleihaltigen monokristallinen Perlglanzpigmenten, wie basisches Bleicarbonat oder Bleihydrogenphosphat, haben insbesondere nichttoxische Perlglanzpigmente verstärktes Interesse hervorgerufen. Beispielsweise können diesbezüglich Pigmente auf Basis von Bismutoxidchlorid genannt werden, die sich durch hohen Glanz und gutes Deckvermögen auszeichnen. Bismutoxidchlorid weist jedoch eine geringe Lichtstabilität auf und kann in Pastenform angeboten werden. Ein weiterer Nachteil ist die geringe mechanische Belastbarkeit.

Die größte Verbreitung haben Perlglanzpigmente, die aus auf einem Substrat aufgebrachten Interferenzschichten bestehen. Die Interferenzschichten bestehen aus Metalloxiden wie beispielsweise Titandioxid oder Eisenoxid. Als Substrat kommen natürlicher oder synthetischer Glimmer, Glasflakes, Aluminiuoxidflakes, Siliciumdioxid oder metallische Flakes in Frage.

Interferenzpigmente auf der Basis von transparenten plättchenförmigen Substraten, wie Glimmer, Siliciumdioxid oder Aluminiumoxid, die mit einer hochbrechenden Beschichtung, bestehend aus TiO₂, belegt sind, werden in EP 1 564 261 A2 beschrieben. Die plättchenförmigen Substrate weisen eine durchschnittliche Dicke zwischen 0,02 und 2 µm, vorzugsweise zwischen 0,1 und 1 µm und besonders bevorzugt zwischen 0,2 und 0,8 µm auf. Zur Erzielung eines intensiven Farbeffekts mit überlagerter, winkelabhängiger Farbtönung muss die durchschnittliche Dicke der einzelnen Plättchen innerhalb einer Standardabweichung von < 15 % liegen.

In WO 2011/095326 A1 werden Effektpigmente auf der Basis von unbeschichteten oder beschichteten plättchenförmigen Substraten beschrieben, welche eine äußere metalloxidhaltige, calcinierte Beschichtung aufweisen, welche verschiedene Metalloxide enthalten. In Bezug auf die Dimensionen der plättchenförmigen Substrate beschreibt WO 2011/095326 A1, dass Substrate mit gewöhnlicher Größe eingesetzt werden können, ohne besondere Anforderungen zu stellen. Aufgrund dieser äußeren Beschichtung weisen diese Effektpigmente auch ohne entsprechende Nachbeschichtungen eine hohe Stabilität auf.

In EP 1 072 651 A1 werden Pigmente auf Basis dünner Flocken mit einer durchschnittlichen Partikelgröße von 0,5 µm bis 10 µm, vorzugsweise 2 µm bis 8 µm, beschrieben, die zunächst mit kugelförmigen SiO₂-Partikeln und nachfolgend mit ultrafeinen TiO₂-Partikeln beschichtet sind. Derartige Pigmente werden u.a. aufgrund ihres Softfokuseffekts als Füllstoffe beispielsweise kosmetischen Formulierungen zugesetzt. Durch die kugelförmige Struktur der SiO₂- und TiO₂-Partikel erfolgt im Wesentlichen eine ungerichtete Reflexion, die in der kosmetischen Applikation auf der Haut einen unerwünschten "whitening-effect" hervorruft.

Eine vergleichbare Anwendung wird für die in WO 2011/045030 A1 offenbarten Perlglanzpigmenten beschrieben. Diese Perlglanzpigmente umfassen ein weitgehend transparentes plättchenförmiges synthetisches Substrat, das mit einer optisch wirksamen Beschichtung umhüllt ist, wobei das Substrat einen außerordentlich geringen Durchmesser und eine geringe Substratdicke mit einer mittleren Dicke im Bereich von 40 nm bis 110 nm aufweist. Durch diese geringe Teilchengröße, welche vorzugsweise in einem Bereich von 3,0 bis 5,0 µm liegt, kann ein unerwünschter Glanz verhindert werden. Aufgrund der geringen Aspect Ratio (Verhältnis zwischen Substratdurchmesser und Dicke), ist der Perlglanzeffekt aber wenig ausgeprägt.

Des Weiteren gibt es noch Perlglanzpigmente, die auf plättchenförmigen metallischen Substraten beruhen. Die Größen der Substratteilchen liegen bei 5 bis 100 µm und die Dicken zwischen 0,1 µm und 5 µm. Aufgrund des metallischen Kerns müssen diese Pigmente jedoch phlegmatisiert werden, beispielweise mit bestimmten Lösemittel, um deren Empfindlichkeit in Bezug auf eine explosive Reaktion zu vermindern.

Die aus dem Stand der Technik bekannten Perlglanzpigmente weisen jedoch in gewisser Hinsicht erhebliche Mängel auf. Grundsätzliche ist es aus Effizienzgründen wünschenswert, Perlglanzpigmente mit hohem Deckvermögen und hoher Chroma bereitzustellen, um den Anforderungen nach immer dünner werdenden Lackschichten, wie beispielsweise in der Automobilindustrie, gerecht zu werden. Ein weiterer Nachteil bekannter Perlglanzpigmente sind die mit den Herstellungsverfahren verbundenen hohen Kosten.

Somit liegt der vorliegenden Erfindung die Aufgabe zugrunde, ein flexibles und kostengünstiges Verfahren zur Herstellung von umweltgerechten Perlglanzpigmenten sowie entsprechende Perlglanzpigmente bereitzustellen, welche ein hohes Deckvermögen, eine gute Stabilität und zugleich eine ausgezeichnete Chroma (Buntheit) aufweisen sollen.

Diese Aufgabe wird durch die in den Ansprüchen gekennzeichneten Ausführungsformen gelöst.

Insbesondere wird ein Verfahren zur Herstellung von Perlglanzpigmenten bereitgestellt, umfassend die Schritte
des Bereitstellens von monolithisch aufgebauten metallischen Substratplättchen mit einer durchschnittlichen Dicke von 1 bis 40 nm und einem Formfaktor, ausgedrückt durch das Verhältnis der mittleren Größe zur durchschnittlichen Dicke, von mindestens 80,
des Beschichtens der metallischen Substratplättchen mit einer umhüllenden im Wesentlichen transparenten Beschichtung A aus mindestens einem niedrigbrechenden Metalloxid und/oder Metalloxid hydrat, das einen Brechungsindex von weniger als 1,8 aufweist,
des Beschichtens der derart beschichteten metallischen Substratplättchen mit mindestens einer Interferenzschicht in Form einer Beschichtung B aus mindestens einem hochbrechenden Metalloxid, das einen Brechungsindex von mindestens 1,8 aufweist,
wobei die Beschichtungsschritte durch hydrolytische Zersetzung einer oder mehrerer organischer Metallverbindungen und/oder durch Fällung eines oder mehrerer gelöster Metallsalze erfolgen, und
anschließend des Calcinierens der derart beschichteten Substratplättchen bei 500 bis 1200°C, wodurch das metallische Substratplättchen in das entsprechende Metalloxid überführt wird.

Durch das erfindungsgemäße Verfahren ist es möglich, auf flexible und kostengünstige Art Perlglanzpigmente herzustellen, welche ein hohes Deckvermögen, eine gute Stabilität und zugleich eine ausgezeichnete Chroma (Buntheit) aufweisen. Insbesondere ist es mit Hilfe des erfindungsgemäßen Herstellungsverfahrens möglich, Perlglanzpigmente in einfacher und flexibler Weise in großen Mengen reproduzierbar herzustellen, die sich durch hohe optische Qualität auszeichnen.

Die durch das erfindungsgemäße Verfahren erzeugten Perlglanzpigmente weisen monolithisch aufgebaute Substratplättchen aus einem Metalloxid mit einer durchschnittlichen Dicke von 1 bis 40 nm und einem Formfaktor (Aspect Ratio des Substrates), ausgedrückt durch das Verhältnis der mittleren Größe zur durchschnittlichen Dicke, von mindestens 80 auf, wobei die Substratplättchen von mindestens einer im Wesentlichen transparenten Beschichtung A aus mindestens einem niedrigbrechenden Metalloxid und/oder Metalloxidhydrat, das einen Brechungsindex von weniger als 1,8 aufweist, umhüllt sind und darauf angeordnet bzw. umhüllend mindestens eine Interferenzschicht in Form einer Beschichtung B aus mindestens einem hochbrechenden Metalloxid, das einen Brechungsindex von mindestens 1,8 aufweisen.

Die erfindungsgemäßen Perlglanzpigmente lassen sich kostengünstig herstellen. Sie weisen ein außergewöhnlich hohes Deckvermögen auf und bieten somit vielfältige Vorteile für deren Verwendung, beispielsweise als Lack in der Automobil- und Fahrzeugindustrie. Die Perlglanzpigmente gemäß der vorliegenden Erfindung zeichnen sich darüber hinaus im Vergleich zu herkömmlichen Perlglanzpigmenten durch eine ausgezeichnete Buntheit aus.

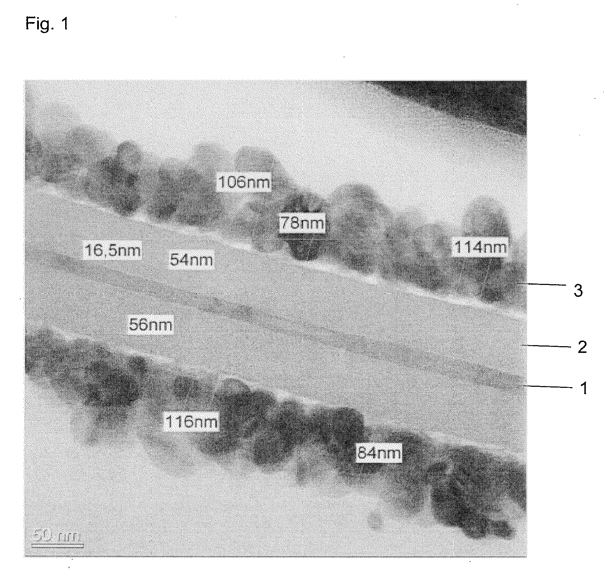

Die Substratplättchen weisen eine durchschnittliche Dicke von höchstens 40 nm, vorzugsweise weniger als 40 nm, besonders bevorzugt höchstens 25 nm, beispielsweise höchstens 20 nm, auf. Die durchschnittliche Dicke der Substratplättchen beträgt mindestens 1 nm, vorzugsweise mindestens 2,5 nm, besonders bevorzugt mindestens 5 nm, beispielsweise mindestens 10 nm. Bevorzugte Bereiche für die Dicke der Substratplättchen sind 2,5 bis 40 nm, 5 bis 40 nm, 10 bis 40 nm, 2,5 bis 30 nm, 5 bis 30 nm, 10 bis 30 nm, 2,5 bis 25 nm, 5 bis 25 nm, 10 bis 25 nm, 2,5 bis 20 nm, 5 bis 20 nm und 10 bis 20 nm. Vorzugsweise weist jedes Substratplättchen eine möglichst einheitliche Dicke auf. Herstellungsbedingt können allerdings Schwankungen der Dicke innerhalb eines Plättchens auftreten. Diese sollten vorzugsweise nicht mehr als ±50%, bezogen auf die durchschnittliche Dicke des betrachteten Plättchens, mehr bevorzugt höchstens ±25%, besonders bevorzugt höchstens ±10%, insbesondere bevorzugt höchstens ±5% betragen. Unter der durchschnittlichen Dicke ist hier das Zahlenmittel aus maximaler und minimaler Dicke zu verstehen. Die Ermittlung der minimalen und maximalen Schichtdicke erfolgt durch Ausmessen auf Grundlage einer transmissionselektronenmikroskopischen (TEM) Aufnahme eines (beschichteten) Substratplättchens (vgl. Figur 1). Nachdem die Farbe der beschichteten Substratplättchen von der Schichtdicke abhängig ist, wird durch eine präzise und einheitlich eingestellte Dicke der unbeschichteten Substratplättchen eine einheitliche Farbwirkung sichergestellt.

Bezüglich der Schwankung der Schichtdicke und der Bestimmung der (durchschnittlichen) Schichtdicke gilt das Vorstehende analog auch für die Dicken der Beschichtungen A sowie B und, falls vorhanden, C.

Soweit hier auf die "Dicke" einer Beschichtung oder eines Substratplättchens Bezug genommen wird, gilt damit die durchschnittliche Dicke als bezeichnet, sofern an betreffender Stelle nichts anderes bestimmt ist.

Die Substratplättchen sind monolithisch aufgebaut. Monolithisch bedeutet in diesem Zusammenhang aus einer einzigen abgeschlossenen Einheit ohne Brüche, Schichtungen oder Einschlüsse bestehend. Dies trifft sowohl für die metallischen Substratplättchen als auch die oxidierten Substratplättchen nach dem Calcinierungsschritt zu. Die oxidierten Substratplättchen sind vorzugsweise homogen aufgebaut, d.h. dass innerhalb der Plättchen im Wesentlichen kein Konzentrationsgradient auftritt. Insbesondere sind die oxidierten Substratplättchen nicht schichtartig aufgebaut und weisen keine darin verteilten Teilchen oder Partikel auf. Sie weisen insbesondere keinen Kern-Schale Aufbau auf, wobei die Schale beispielsweise aus einem für die Substratplättchen geeigneten Material und der Kern aus einem anderen Material, beispielsweise einem Siliciumoxid, besteht. Durch ihren einfachen Aufbau sind die Substratplättchen kostengünstig und effizient herzustellen. Im Gegensatz dazu bedingt ein komplexerer, nicht-monolithischer Aufbau der Substratplättchen einen erschwerten, zeit- und kostenintensiven Herstellungsprozess.

Der Massenanteil des Substratplättchens an dem beschichteten Substratplättchen beträgt vorzugsweise höchstens 25 Gew.-%, besonders bevorzugt höchstens 20 Gew.-%, beispielsweise höchstens 15 Gew.-%. Allerdings sollte der Massenanteil der Substratplättchen nicht unterhalb von 1 Gew.-%, vorzugsweise nicht unter 2 Gew.-% oder 3 Gew.-% fallen. Besonders bevorzugt beträgt der Massenanteil des Substratplättchens an dem beschichteten Substratplättchen 5 bis 12 Gew.-%.

Durch die geringe Dicke bzw. den geringen Massenanteil der Substratplättchen weist das erfindungsgemäße Perlglanzpigment ein besonders hohes Deckvermögen auf. Darüber hinaus können aufgrund der geringen Dicke/des geringen Massenanteils der Substratplättchen auch teure und seltene Materialien wie spezielle Übergangsmetalle ressourcenschonend und wirtschaftlich als Material für die Substratplättchen verwendet werden.

Abgesehen von der Dicke ist die Größe der unbeschichteten Substratplättchen dadurch gekennzeichnet, dass der Formfaktor (Aspect Ratio), ausgedrückt durch das Verhältnis der mittleren Größe zur durchschnittlichen Dicke, mindestens 80, vorzugsweise mindestens 200, mehr bevorzugt mindestens 500, besonders bevorzugt mehr als 750, beträgt. Dabei wird gemäß der vorliegenden Erfindung als mittlere Größe der unbeschichteten Substratplättchen der d₅₀-Wert der unbeschichteten Substratplättchen verstanden. Hierin wird der d₅₀-Wert, soweit nicht anders angegeben, mit einem Gerät des Typs Sympatec Helos mit Quixel-Nassdispergierung bestimmt. Dabei wird zur Probenvorbereitung die zu untersuchende Probe für eine Dauer von 3 Minuten in Isopropanol vordispergiert.

Dementsprechend ist die Größe der unbeschichteten Substratplättchen nicht kritisch und kann auf den jeweiligen Anwendungszweck abgestimmt werden, insofern der Formfaktor mindestens 80 beträgt. Beispielsweise beträgt die mittlere Größe d₅₀ der unbeschichteten Substratplättchen etwa 2 bis 200 µm. Gemäß einer bevorzugten Ausführungsform beträgt die mittlere Größe d₅₀ der unbeschichteten Substratplättchen 5 bis 100 µm, wobei die Untergrenze mehr bevorzugt mindestens 8 µm und besonders bevorzugt mindestens 10 µm beträgt. Die Obergrenze beträgt gemäß einer besonders bevorzugten Ausführungsform 50 µm, insbesondere 30 µm. Die mittlere Größe d₅₀ der unbeschichteten Substratplättchen liegt insbesondere zwischen 2 und 50 µm, besonders bevorzugt zwischen 5 und 30 µm. Allerdings kann die mittlere Größe d₅₀ der unbeschichteten Substratplättchen je nach Verwendungszweck, beispielsweise als Industrielack, beispielweise bei Werten von etwa 70 µm liegen. Für den Fall, dass die mittlere Größe d₅₀ der unbeschichteten Substratplättchen etwa 2 µm beträgt, ist es für einen Fachmann klar, eine entsprechend niedrigere Obergrenze für die durchschnittliche Dicke der unbeschichteten Substratplättchen zu wählen (hier: 25 nm). Gleiches gilt für die entsprechenden bevorzugten Untergrenzen.

Die beschichteten Substratplättchen weisen vorzugsweise eine Gesamtdicke von 50 bis 800 nm, besonders bevorzugt 100 bis 700 nm, insbesondere bevorzugt 130 bis 400 nm, beispielsweise 150 bis 350 nm, auf. Aufgrund der geringen Dicke der Substratplättchen weist das erfindungsgemäße Perlglanzpigment ein besonders hohes Deckvermögen auf. Die geringe Gesamtdicke der beschichteten Substratplättchen wird insbesondere dadurch erzielt, dass die Dicke der unbeschichteten Substratplättchen gering ist, aber auch dadurch, dass die Dicken der Beschichtungen A und, falls vorhanden, C auf einen möglichst kleinen Wert eingestellt werden. Nachdem die Dicke von Beschichtung B den Farbeindruck des Perlglanzpigments bestimmt, ist bei einem festgelegten gewünschten Farbeffekt diesbezüglich kein Spielraum gegeben.

Bisher wurde davon ausgegangen, dass im Wesentlichen nicht lichtdurchlässige (opake) Materialien, wie beispielsweise Metalle, als Substratplättchen geeignet sind, um eine hohes Deckvermögen zu erzielen. Zudem war es technisch nicht möglich anorganische Substrate mit geringen Schichtdicken (unter 200 nm) herzustellen. Darüber hinaus wurde angenommen, dass unter anderem wegen der Bruchgefahr, was zu einem stark herabgesetztem Deckvermögen des resultierenden Glanzpigments führen würde, eine gewisse Dicke nicht unterschreiten werden dürfte.

Allerdings konnte festgestellt werden, dass mit (teilweise oder ganz lichtdurchlässigen) Substratplättchen mit einer Schichtdicke von höchstens 40 nm, vorzugsweise höchstens 25 nm, Perlglanzpigmente hergestellt werden können, die ein deutlich höheres Deckvermögen aufweisen als gewöhnliche metallische Glanzpigmente. Wahrscheinlich liegt der Grund dafür darin, dass durch die geringe Gesamtdicke der beschichteten Substratplättchen eine höhere Flächenabdeckung des Perlglanzpigments erzielt wird. Nachdem die beschichteten Substratplättchen dünn sind, kann mit der gleichen Masse Pigment eine höhere Fläche abgedeckt werden. Durch diesen vorteilhaften Effekt wird die höhere Lichtdurchlässigkeit dünner, ganz oder teilweise lichtdurchlässiger Substratplättchen überkompensiert, so dass letztendlich gegenüber Perlglanzpigmenten mit dicken Substratplättchen ein höheres Deckvermögen und/oder eine höhere Chroma erzielt wird.

Gemäß der vorliegenden Erfindung werden für die Herstellung der Perlglanzpigmente metallische Substratplättchen verwendet. Als Metall kommt jedes Metall und Halbmetall sowie Metalllegierung in Betracht, welches im erfindungsgemäßen Calcinierungsschritt in ein entsprechendes Metalloxid überführt werden kann. Derartige Metalle sind unter anderem (Übergangs-)metalle wie beispielsweise Platin, Zink, Chrom, Molybdän, aber auch Halbmetalle wie beispielsweise Silicium, sowie deren Legierungen. Bevorzugte Metalle sind Aluminium, Kupfer, Zink und Silicium. Bevorzugte Substratplättchen sind Aluminiumplättchen, Kupferplättchen und Messingplättchen, wobei Aluminiumplättchen besonders bevorzugt sind. Um die Oxidierung der metallischen Substratplättchen im Calcinierungsschritt zu fördern, können die Metallplättchen gegebenenfalls durch geeignete Maßnahmen, wie z.B. Dotierung, behandelt werden.

Aluminiumplättchen können unter anderem durch Herausstanzen aus Aluminiumfolie oder nach gängigen Mahl- und Verdüsungstechniken hergestellt werden. So sind beispielsweise Aluminiumplättchen aus dem Hallverfahren, einem Nassmahlverfahren, erhältlich. Andere Metallplättchen, beispielsweise aus Bronze, können in einem Trockenmahlverfahren wie dem Hametagverfahren erhalten werden.

Die Aluminium- oder Metallplättchen können verschiedene Formen aufweisen. Als Substratplättchen können beispielsweise lamellare und lenticulare Metallplättchen oder auch sog. *vacuum metallized pigments* (VMP) verwendet werden. Lamellare Metallplättchen zeichnen sich durch einen unregelmäßig strukturierten Rand aus und werden aufgrund ihres Erscheinungsbildes auch als "cornflakes" bezeichnet. Lenticulare Metallplättchen weisen einen im Wesentlichen regelmäßigen runden Rand auf und werden aufgrund ihres Erscheinungsbildes auch als "silverdollars" bezeichnet. Aufgrund ihrer unregelmäßigen Struktur erzeugen metallische Glanzpigmente auf der Basis von lamellaren Metallplättchen einen höheren Anteil an Streulicht als lenticulare Metallplättchen, wohingegen bei letztgenannten der Anteil des reflektierten Lichts überwiegt.

Vorzugsweise werden erfindungsgemäß VMPs verwendet. VMPs können durch das Freisetzen von Aluminium von metallisierten Folien gewonnen werden. Sie zeichnen sich durch eine besonders geringe Dicke der Substratplättchen im Bereich von 1 bis 40 nm und durch eine besonders glatte Oberfläche mit erhöhter Reflektivität aus.

Gemäß der vorliegenden Erfindung wird das metallische Substratplättchen im Calcinierungsschritt im Wesentlichen vollständig in das entsprechende Metalloxid überführt, weshalb das Substratplättchen im erfindungsgemäßen Perlglanzpigment als monolithisches und homogenes Material vorliegt. Dadurch unterscheidet sich das erfindungsgemäße Herstellungsverfahren von gewöhnlichen Verfahren, bei welchen die Calcinierungsprozesse lediglich zum Brennen der äußeren Schicht(en) führt. Wie vorstehend angemerkt, weist das erfindungsgemäß erhaltene Perlglanzpigment keine Substratplättchen auf, bei denen lediglich eine Oxidschicht beispielsweise in Form einer Passivierung auf dem metallischen Kern gebildet ist.

Hier bedeutet der Begriff "im Wesentlichen", sofern er auf einen Bestandteil einer Zusammensetzung angewandt wird, dass die Zusammensetzung zu mindestens 95 Gew.-%, bevorzugt zu mindestens 99 Gew.-%, insbesondere bevorzugt zu mindestens 99,5 Gew.-%, beispielsweise zu etwa 100 Gew.-% aus dem bezeichneten Bestandteil aufgebaut ist.

Dass gemäß der vorliegenden Erfindung das metallische Substratplättchen in das entsprechende Metalloxid überführt wird, liegt insbesondere in der geringen Dicke des Substratplättchens begründet. Falls die Dicke der Substratplättchen größer als 200 nm sein sollte, ist es schwierig, eine vollständige Oxidation des metallischen Substratplättchens im Rahmen des Calcinierungsschritts zu erhalten. Die Beurteilung, ob das metallische Substratplättchen in das entsprechende Metalloxid überführt ist, kann vorzugsweise mittels transmissionselektronenmikroskopischen (TEM) Aufnahmen in Kombination mit energiedispersiver Röntgenanalyse ermittelt werden.

Erfindungsgemäß sind die beschichteten Substratplättchen von mindestens einer Interferenzschicht in Form einer Beschichtung B aus einem hochbrechenden Metalloxid umhüllt. Zwischen der Beschichtung B und der Substratoberfläche weisen die beschichteten Substratplättchen eine umhüllende im Wesentlichen transparente Beschichtung A auf. Gegebenenfalls weisen die Substratplättchen eine weitere Beschichtung C, die von der darunterliegenden Schicht B verschieden ist, auf.

Als Materialien für die Beschichtungen A, B und C eignen sich alle Substanzen, die filmartig und dauerhaft auf die Substratplättchen aufgebracht werden können und, im Fall der Schichten A und B, die erforderlichen optischen Eigenschaften aufweisen. In diesem Zusammenhang bedeutet der Begriff "im Wesentlichen transparent", dass die Beschichtungen A mindestens 70%, bevorzugt mindestens 80%, insbesondere bevorzugt mindestens 90%, des einfallenden sichtbaren Lichts durchlassen.

Allgemein ist eine Beschichtung eines Teils der Oberfläche der beschichteten Substratplättchen ausreichend, um ein Perlglanzpigment zu erhalten. So können beispielsweise lediglich die obere und/oder untere Seite der Plättchen beschichtet sein, wobei die Seitenfläche(n) ausgespart sind. Erfindungsgemäß ist allerdings die gesamte Oberfläche der Substratplättchen, einschließlich der Seitenflächen, von Beschichtung B bedeckt. Die Substratplättchen sind also vollständig von Beschichtung B umhüllt. Dies verbessert die optischen Eigenschaften des erfindungsgemäßen Pigments und erhöht die mechanische und chemische Belastbarkeit der beschichteten Substratplättchen. Das Vorstehende gilt auch für die Schicht A und vorzugsweise auch für die Schicht C, falls vorhanden.

Obwohl jeweils mehrere Beschichtungen A, B und/oder C vorhanden sein können, weisen die beschichteten Substratplättchen vorzugsweise jeweils nur eine Beschichtung A, B und, falls vorhanden, C auf. Gemäß einer weiteren bevorzugten Ausführungsform können die erfindungsgemäßen Perlglanzpigmente jedoch zwei Interferenzschichten in Form der Beschichtung B aufweisen, beispielsweise eine Kombination von Eisenoxid (Fe₂O₃) und TiO₂.

Die Beschichtung B ist aus mindestens einem hochbrechenden Metalloxid aufgebaut. Vorzugsweise umfasst die Beschichtung B mindestens 95 Gew.-%, besonders bevorzugt mindestens 99 Gew.-%, beispielsweise etwa 100 Gew.-% mindestens, eines hochbrechenden Metalloxids.

Gemäß einer bevorzugten Ausführungsform weist die Interferenzschicht in Form der Beschichtung B eine Dicke von mindestens 20 nm, mehr bevorzugt mindestens 40 nm, besonders bevorzugt mindestens 50 nm, auf. Vorzugsweise beträgt die Dicke von Beschichtung B nicht mehr als 250 nm, besonders bevorzugt höchstens 150 nm. Die Dicke der Interferenzschicht bedeutet in diesem Zusammenhang die einfache Schichtdicke der Beschichtung B, welche aufgrund der Umhüllung im erfindungsgemäßen Perlglanzpigment entsprechend doppelt eingeht.

Das Verhältnis der Dicke von Beschichtung B zur Dicke der unbeschichteten Substratplättchen beträgt vorzugsweise mindestens 2, beispielsweise 4, 8 oder 10. Grundsätzlich muss für dieses Verhältnis keine Obergrenze eingehalten werden, es sollte aber aus praktischen Gründen höchstens 1000, vorzugsweise höchstens 500, betragen. Die durchschnittliche Dicke einer Beschichtung beziehungsweise eines Substratplättchens bestimmt sich aus dem arithmetischen Mittel der maximalen und minimalen Dicke der Beschichtung/des Substratplättchens.

Soweit hier auf "Substratplättchen", ohne Unterscheidung, ob diese beschichtet sind oder nicht, Bezug genommen wird, gelten damit unbeschichtete Substratplättchen als bezeichnet, sofern an betreffender Stelle nichts anderes bestimmt ist.

Erfindungsgemäß ist zwischen der Oberfläche der Substratplättchen und der Beschichtung B eine weitere Beschichtung A aus mindestens einem niedrigbrechenden Metalloxid(hydrat) vorhanden. Gemäß der vorliegenden Erfindung kann die Beschichtung A als eine einzelne Beschichtung vorliegen. Es können jedoch auch zwei oder mehrere im Wesentlichen transparente Beschichtungen A zwischen der Beschichtung B und dem Substratplättchen angeordnet sein. Die Beschichtung A ist aus mindestens einem niedrigbrechendem Metalloxid und/oder Metalloxidhydrat aufgebaut. Vorzugsweise umfasst Beschichtung A mindestens 95 Gew.-%, besonders bevorzugt mindestens 99 Gew.-%, beispielsweise etwa 100 Gew.-% eines niedrigbrechenden Metalloxid(hydrat)s.

Gelegentlich weisen die Metalloxide, welche für die Beschichtungen A, B und C eingesetzt werden können, einen gewissen Anteil an Nebenbestandteilen und/oder Verunreinigungen auf. Zu typischen Nebenbestandteilen von Metalloxiden zählen insbesondere Metallhydroxide. So kann beispielsweise eine Beschichtung aus Eisenoxid einen gewissen Anteil an Eisenhydroxid enthalten.

Hier bezeichnen die Begriffe "hochbrechend" und "niedrigbrechend" jeweils Materialien mit einem hohen bzw. niedrigen Brechungsindex. Hochbrechende Materialien weisen einen Brechungsindex von mindestens 1,8, bevorzugt mindestens 2,0 und besonders bevorzugt mindestens 2,4 auf. Niedrigbrechende Materialien weisen einen Brechungsindex von weniger als 1,8, bevorzugt höchstens 1,6 auf.

Für Beschichtung B geeignete hochbrechende Metalloxide sind vorzugsweise selektiv lichtabsorbierende (d.h. farbige) Metalloxide, wie beispielsweise Eisen(III)oxid (α- und γ-Fe₂O₃, rot), Cobalt(II)oxid (blau), Chrom(III)oxid (grün), Titan(III)oxid (blau, liegt üblicherweise im Gemisch mit Titanoxynitriden und Titannitriden vor), Nickel(II)oxid (grünlich), Kupfer(I/II)oxid (blau) und Vanadium(V)oxid (orange) sowie deren Gemische, ohne jedoch auf diese beschränkt zu sein. Es eignen sich auch farblose hochbrechende Oxide wie Titandioxid, Antimon(III)oxid, Zink(II)oxid und/oder Zirkoniumdioxid. Vorzugsweise besteht die Interferenzschicht im Wesentlichen aus Titandioxid oder, im Falle von farbigen Metalloxiden, aus Eisen(III)oxid.

Darüber hinaus ist es bevorzugt, dass die Interferenzschicht im Falle von farblosen Oxiden entsprechende Dotierungsmittel enthält. Beispielsweise ist es bevorzugt, wenn die Beschichtung B aus Titandioxid Dotierungsstoffe, ausgewählt aus Zinn, Aluminium, Lithium, Zirkonium, Eisen oder Cer, insbesondere deren Salze enthält, vorzugsweise 0,001 bis 5 Gew.-%, besonders bevorzugt 0,01 bis 1 Gew.-%. Zudem kann die Interferenzschicht einen selektiv absorbierenden Farbstoff enthalten, vorzugsweise 0,001 bis 5 Gew.-%, besonders bevorzugt 0,01 bis 1 Gew.-%. Geeignet sind organische und anorganische Farbstoffe, die sich stabil in eine Metalloxidbeschichtung einbauen lassen.

Zur Rutilisierung von Titandioxid kann ferner eine Zinndioxidschicht unter der Titandioxidschicht aufgebracht sein. Dementsprechend kann im erfindungsgemäßen Perlglanzpigment eine dünne nur wenige nm dicke (< 10 nm) Zinndioxidschicht zwischen der Beschichtung A und der Interferenzschicht enthalten sein.

Zu den niedrigbrechenden Metalloxiden, die für die Beschichtung A geeignet sind, zählen beispielsweise Silicium(di)oxid, Siliciumoxidhydrat, Aluminiumoxid, Aluminiumoxidhydrat, Ceroxid, Zinnoxid (SnO₂) und deren Gemische, wobei Siliciumdioxid bevorzugt ist. Die Dicke der Beschichtung A beträgt gewöhnlich 1 bis 1000 nm, vorzugsweise 5 bis 300 nm, besonders bevorzugt 10 bis 150 nm. Gemäß einer bevorzugten Ausführungsform weist die Beschichtung A eine Dicke von 5 bis 50 nm, besonders bevorzugt 5 bis 40 nm, insbesondere bevorzugt 10 bis 40 nm, auf.

Wie nachfolgend genauer beschrieben, kann erfindungsgemäß die Dicke der Beschichtung A, wie auch die Dicke der Beschichtung B, variabel und präzise eingestellt werden.

In vorstehend beschriebener Ausführungsform, bei welcher die Beschichtung A eine Dicke von 5 bis 50 nm aufweist, beträgt der Abstand zwischen der Oberfläche der Substratplättchen und der inneren Oberfläche von Beschichtung B vorzugsweise höchstens 100 nm, besonders bevorzugt höchstens 50 nm, insbesondere bevorzugt höchstens 20 nm. Dadurch, dass die Dicke von Beschichtung A/der Abstand zwischen der Oberfläche der Substratplättchen und Beschichtung B im oben angegebenen Bereich liegt, kann sichergestellt werden, dass die beschichteten Substratplättchen des erfindungsgemäßen Perlglanzpigments im Falle von farbigen Oxiden ein hohes Deckvermögen sowie einen kleinen ΔE-Wert aufweisen. Der ΔE-Wert ist der Gesamtfarbabstand, der bei den erfindungsgemäßen Perlglanzpigmenten vorzugsweise höchstens 25, besonders bevorzugt höchstens 20, insbesondere höchstens 15 beträgt. Die Messung von ΔE erfolgt hierbei nach DIN 55987, wobei auf jeweils eine schwarze und eine weiße Oberfläche eine Lackschicht, die das erfindungsgemäße Perlglanzpigment mit einem Massenanteil von 12 Gew.-% (Trockengewicht) enthält, aufgetragen wird. Die Schichtdicke der getrockneten Lackierung beträgt 12 µm. Danach wird der Gesamtfarbabstand ΔE zwischen den Lackierungen auf weißem und schwarzem Grund bestimmt. Im Fälle einer transparenten Beschichtung B (zum Beispiel bei Verwendung von TiO₂) führt die geringe Dicke des Substrates zu einer hohen Chroma im Interferenzwinkel (15° vom Glanz)

Gemäß einer weiteren bevorzugten Ausführungsform beträgt die Dicke der Beschichtung A mehr als 50 nm. Wenn die Schichtdicke der Beschichtung A mehr als 50 nm beträgt, kann im erfindungsgemäßen Perlglanzpigment ein zusätzlicher Interferenzeffekt erzeugt werden. Die Dicke der Beschichtung A kann hierzu entsprechend den Materialien und Dimensionen des Substratplättchens und der weiteren Beschichtungen variabel und exakt eingestellt werden, wobei als bevorzugte Obergrenze der Dicke die vorstehend genannten Werte gelten. Der zusätzliche Interferenzeffekt kann darüber hinaus durch die Verwendung von mindestens einer auf die Dicke der Beschichtung A abgestimmten Interferenzschicht unterstützt werden, wobei auch zwei oder mehr dieser Interferenzschichten vorhanden sein können. Insbesondere in einem Bereich von 80 bis 200 nm für die Schichtdicke der Beschichtung A können in Kombination mit der Interferenzschicht B besondere Farbeffekte erzielt werden.

Erfindungsgemäß können die Perlglanzpigmente eine weitere Beschichtung C aufweisen, wodurch bestimmte Oberflächeneigenschaften gezielt eingestellt werden können. Diese Beschichtung C ist gemäß einer bevorzugten Ausführungsform aus einem Metalloxid(hydrat), das von der darunterliegenden Beschichtung B verschieden ist, oder aus organischen Silanverbindungen ausgewählt. Beispiele für Organosilane sind Propyltrimethoxysilan, Propyltriethoxysilan, Isobutyltrimethoxysilan, n-Octyltrimethoxysilan, i-Octyltrimethoxysilan, n-Octyltriethoxysilan, n-Decyltrimethoxysilan, Dodecyltrimethoxysilan, Hexadecyltrimethoxysilan, Vinyltrimethoxysilan.

Geeignete Metalloxid(hydrate) sind beispielsweise Silicium(di)oxid, Siliciumoxidhydrat, Aluminiumoxid, Aluminiumoxidhydrat, Zinkoxid, Zinnoxid, Titandioxid, Zirkonoxid, Eisen(III)oxid und Chrom(III)oxid. Bevorzugt ist Siliciumdioxid.

Die Beschichtung C weist vorzugsweise eine Dicke von 1 bis 500 nm, mehr bevorzugt von 10 bis 500 nm, besonders bevorzugt 50 bis 300 nm auf.

Obwohl jede der Beschichtungen A, B und/oder C aus einem Gemisch von zwei oder mehreren Metalloxid(hydrat)en aufgebaut sein kann, ist jede der Beschichtungen vorzugsweise aus einem Metalloxid(hydrat) aufgebaut.

Wenn Aluminium als metallisches Substratplättchenmaterial eingesetzt wird, beträgt vor dem Calcinierungsschritt das Stoffmengenverhältnis α von Sauerstoff, der nicht an Aluminium gebunden ist, zu Aluminium vorzugsweise mindestens 3, besonders bevorzugt mindestens 4, insbesondere bevorzugt mindestens 5. Wenn α mindestens 3 beträgt, wird vermieden, dass in den beschichteten Substratplättchen ein Stoffmengenverhältnis von Sauerstoff, der nicht an Aluminium gebunden ist, zu Aluminium im stöchiometrischen Verhältnis von 3/2 (mol/mol) vorliegt. Ein Gemisch von Aluminium und Sauerstoffverbindungen, insbesondere Fe₂O₃, in dem das Stoffmengenverhältnis α im Bereich von 3/2 liegt, kann aufgrund der hohen Oxophilie von Aluminiummetall stark exotherm reagieren, unter Umständen mit Explosionswirkung (Aluminothermie, Thermitreaktion). Daher kann ein Gemisch mit α im Bereich von 3/2 eine Sicherheitsgefährdung darstellen. Die Reaktionsfähigkeit eines derartigen Gemisches kann jedoch dadurch herabgesetzt werden, dass das Verhältnis α auf einen Wert, der entweder deutlich größer oder deutlich kleiner als 3/2 ist, eingestellt wird.

Wie vorstehend beschrieben, umfasst das erfindungsgemäße Verfahren zur Herstellung der Perlglanzpigmente den Schritt des Beschichtens der metallischen Substratplättchen durch hydrolytische Zersetzung einer oder mehrerer organischer Metallverbindungen und/oder durch Fällung eines oder mehrerer gelöster Metallsalze.

Zur Erzeugung von Beschichtung A werden zweckmäßigerweise organische Metallverbindungen (vorzugsweise organische Silicium- und/oder Aluminiumverbindungen), bei denen die organischen Reste über Sauerstoffatome an die Metalle gebunden sind, in Gegenwart der metallischen Substratplättchen und eines organischen Lösemittels, in welchem die Metallverbindungen löslich sind, hydrolysiert. Hierfür ist eine Vielzahl von organischen Lösemitteln geeignet, bevorzugt ist iso-Propanol.

Bevorzugte Beispiele für die organischen Metallverbindungen sind die Acetylacetonate und insbesondere Alkoholate, vor allem C₁-C₄-Alkanolate, z.B. Aluminiumtriisopropanolat und Tetraethoxysilan (Tetraethylorthosilikat, TEOS).

Die Hydrolyse wird vorzugsweise in Gegenwart einer Base oder einer Säure als Katalysator durchgeführt. Hierfür eignen sich z. B. neben Alkalilaugen wie Natronlauge insbesondere wässrige Ammoniaklösungen. Geeignete saure Katalysatoren sind beispielsweise Phosphorsäure und organische Säuren wie Essigsäure und Oxalsäure (auch Sol-Gel-Verfahren genannt).

Wasser muss mindestens in der stöchiometrisch für die Hydrolyse erforderlichen Menge vorliegen, bevorzugt ist jedoch die 2 bis 100-fache, insbesondere die 5 bis 20-fache Menge. Bezogen auf die eingesetzte Wassermenge werden vorzugsweise 3 bis 40 Vol.-%, mehr bevorzugt 5 bis 30 Vol.-%, einer 25 Gew.-%igen wässrigen Ammoniaklösung hinzugegeben.

Für die Temperaturführung hat es sich als vorteilhaft erwiesen, das Reaktionsgemisch innerhalb von 10 bis 48 h schrittweise auf Rückflusstemperatur zu erhitzen. Bei Verwendung von iso-Propanol als Lösungsmittel wird das Gemisch zum Beispiel bevorzugt zunächst 4 bis 20 h bei 40°C, dann 4 bis 20 h bei 60°C und zum Schluss 2 bis 8 h bei 80°C gerührt.

Verfahrenstechnisch erfolgt das Beschichten von Substratplättchen mit einer Beschichtung A zweckmäßigerweise wie folgt:
Substratplättchen, organisches Lösungsmittel, Wasser und Katalysator (Säure oder bevorzugt Base, insbesondere z.B. eine wässrige Ammoniaklösung) werden vorgelegt, wonach die zu hydrolysierende Metallverbindung, als Reinstoff oder gelöst, z.B. als 30 bis 70, bevorzugt 40 bis 60 Vol.-%ige Lösung im organischen Lösemittel, zugegeben wird. Erfolgt die Zugabe der Metallverbindung in einem Schritt, dann wird die Suspension anschließend wie vorstehend beschrieben unter Rühren erhitzt. Die Metallverbindung kann aber auch bei erhöhter Temperatur kontinuierlich zudosiert werden, wobei Wasser und Ammoniak vorgelegt oder ebenfalls kontinuierlich zudosiert werden können. Nach beendeter Beschichtung wird das Reaktionsgemisch wieder auf Raumtemperatur abgekühlt.

Um eine Agglomeratbildung während des Beschichtungsvorgangs zu verhindern, kann die Suspension einer starken mechanischen Beanspruchung wie Pumpen, kräftigem Rühren oder Einwirken von Ultraschall unterzogen werden.

Gegebenenfalls kann der Beschichtungsschritt ein- oder mehrfach wiederholt werden. Sollte die Mutterlauge milchig trüb aussehen, so empfiehlt es sich, diese vor einer weiteren Beschichtung auszutauschen.

Die Isolierung der mit Beschichtung A ummantelten metallischen Substratplättchen kann in einfacher Weise durch Abfiltrieren, Waschen mit organischem Lösungsmittel, vorzugsweise den auch als Lösemittel verwendeten Alkoholen, und anschließendes Trocknen (üblicherweise 2 bis 24 h bei 20 bis 200°C) erfolgen. Erfindungsgemäß müssen die mit Beschichtung A ummantelten metallischen Substratplättchen jedoch nicht isoliert werden und können *in situ* für den weiteren Beschichtungsschritt verwendet werden.

Zum Aufbringen der Metalloxidschichten B können beispielsweise α-Eisenoxid- und Chromoxidschichten durch hydrolytische Zersetzung von Eisen(III)salzen wie Eisen (III)-chlorid und -sulfat bzw. Chrom(III)chlorid und anschließendes Überführen der gebildeten hydroxidhaltigen Schichten durch Tempern und/oder Calcinieren in die Oxidschichten aufgebracht werden. In analoger Weise können beispielsweise TiO₂-Schichten mittels TiOCl aufgebracht werden. Ebenso kann auch eine Titan(III)oxidbeschichtung durch Hydrolyse von Titantetrachlorid und anschließende Reduktion des gebildeten Titandioxids mit gasförmigem Ammoniak erreicht werden.

Wenn als Metalloxidschicht B eine α-Eisenoxidschicht vorgesehen wird, kann alternativ dies auch durch Gasphasenzersetzung von flüchtigem Eisenpentacarbonyl in Gegenwart von Sauerstoff (oxidative Zersetzung von Fe(CO)₅) erfolgen. Bei entsprechender Verfahrensführung kann dann in dieser spezifischen Ausführungsform die oxidative Zersetzung auch die Calcinierung der Substratplättchen, also beispielsweise die Überführung der Al-Substratplättchen in Al₂O₃-Substratplättchen, bewirken.

Falls eine Beschichtung C erwünscht ist, kann diese wie für die Beschichtungen A und B beschrieben aufgebracht werden.

Da das Sol-Gel-Verfahren auch in Kesseln mit variabler Größe möglich ist, kann eine hohe Produktvielfalt bei geringen Kosten realisiert werden. Dies ist ein weiterer Vorteil im Vergleich zu konventionellen Bandbeschichtungen, bei welchen die Batchgrößen verfahrensbedingt größer sind.

Gemäß der vorliegenden Erfindung erfolgt in einem weiteren Calcinierungsschritt die Überführung der metallischen Substratplättchen in das entsprechende Metalloxid. Dies erfolgt bei Temperaturen von 500 bis 1200°C. Bei diesen Bedingungen werden die metallischen Substratplättchen vollständig oxidiert, wodurch Perlglanzpigmente mit außergewöhnlich hohem Deckvermögen (im Falle von absorbierenden Interferenzschichten) und Chroma (Buntheit) erhalten werden. Durch die kontrollierte Calcinierung sowie den vorstehend beschriebenen Verfahrensschritten ist es möglich, extrem dünne anorganische Substrate mit verbesserten optischen Eigenschaften bereitzustellen.

Die Calcinierung wird typischer Weise für 30 bis 200 min im vorstehenden Temperaturbereich durchgeführt. Der Calcinierungsschritt erfolgt in einer sauerstoffhaltigen Atmosphäre, wobei keine besonderen Anforderungen an diese gestellt werden. Beispielsweise kann Luft als sauerstoffhaltige Atmosphäre verwendet werden. Darüber hinaus ist es möglich, den Calcinierungsschritt in einer Atmosphäre mit einem Sauerstoffanteil von beispielsweise mindestens 10 Vol.-% durchzuführen.

Überraschenderweise weisen die erfindungsgemäßen Perlglanzpigmente eine ausgezeichnete Chroma (Buntheit) auf. Der Grund hierfür liegt womöglich in der Tatsache, dass der Anteil der Interferenzschicht am Gesamtgewicht der Pigmente sehr viel größer ist als bei konventionellen Pigmenten mit wesentlich dickeren Substraten. Darüber hinaus ist es gemäß der vorliegenden Erfindung möglich, Perlglanzpigmente mit vorstehend beschriebenen Eigenschaften zu erhalten, wobei sowohl die Substratdicke präzise einstellbar ist als auch die weiteren Beschichtungen mit präzisen Dicken auf das Substrat aufgebracht werden können.

Zur Beurteilung der koloristischen Eigenschaften der Perlglanzpigmente können winkelabhängige Helligkeitsmessungen herangezogen werden, wie dies nachstehend detaillierter beschrieben wird. Herbei werden bei konstantem Einfallswinkel von 45° die Farbdaten in Abhängigkeit vom Beobachtungswinkel ermittelt. Zur Bestimmung der koloristischen Eigenschaften der Perlglanzpigmente wird ein Lack mit einer Pigmentkonzentration von 6 oder 12 Gew.-% bezogen auf das Bindemittel hergestellt und mit Hilfe eines Pakels auf eine schwarze Platte mit einer Schichtdicke von 12 µm beschichtet. Die Farbdaten werden mit einem Byk-mac der Firma Byk Additive und Instrumente ermittelt. Es werden die Winkel 15°, 25°, 45°, 75° und 110° vom Glanz abgelesen.

Wie vorstehend beschrieben, können durch die verschiedenen Verfahrensschritte die Dicke der metallischen Substratplättchen und damit verbunden auch die Dicke des nichtmetallischen Substratplättchens im erfindungsgemäßen Perlglanzpigment genau eingestellt werden. Darüber hinaus können die weiteren Beschichtungen mit präzisen Dicken auf das Substrat aufgebracht werden, wodurch echte optische Mehrschichtsysteme erhalten werden.

Da insbesondere weder Aluminiumoxid noch Siliciumdioxid eine Eigenfarbe haben und die erfindungsgemäßen Verfahrensschritte das Auftreten von Verunreinigungen minimiert, ist es möglich Perlglanzpigmente mit reinerem Interferenzeffekt zu erhalten. Das ist insbesondere ein Vorteil gegenüber glimmerbasierten Perlglanzpigmenten.

Mit Hilfe des erfindungsgemäßen Herstellungsverfahrens können die beschichteten Substratplättchen einerseits in einfacher Weise in großen Mengen reproduzierbar hergestellt werden. Andererseits können vollständig umhüllte Pigmentteilchen mit sehr hoher Qualität der einzelnen Beschichtungen (homogen, filmartig) erhalten werden.

Darüber hinaus betrifft die vorliegende Erfindung in einem weiteren Aspekt die Verwendung der vorstehend beschriebenen Perlglanzpigmente zur Einfärbung von Lacken, Druckfarben, Tinten, Kunststoffen, Gläsern, keramischen Produkten und Zubereitungen der dekorativen Kosmetik.

Die erfindungsgemäßen Perlglanzpigmente eignen sich vorteilhaft für viele Zwecke, wie zur Einfärbung von Kunststoffen, Gläsern, keramischen Produkten, Zubereitungen der dekorativen Kosmetik und insbesondere von Tinten, Druckfarben und Sicherheitsdruckfarben und vor allem von Lacken, beispielsweise für die Automobilindustrie.

Für diese Anwendungszwecke lassen sich die erfindungsgemäßen Perlglanzpigmente auch vorteilhaft in Abmischung mit transparenten und deckenden Weiss-, Bunt- und Schwarzpigmenten sowie auch herkömmlichen Glanzpigmenten auf der Basis von metalloxidbeschichteten Glimmer- und Metallpigmenten und plättchenförmigen Eisenoxiden verwenden.

Die erfindungsgemäßen Perlglanzpigmente sind kostengünstig herzustellen. Sie weisen ein außergewöhnlich hohes Deckvermögen sowie eine ausgezeichnete Buntheit auf und bieten somit vielfältige Vorteile für deren Verwendung, beispielsweise als Lack in der Automobil- und Fahrzeugindustrie.
Figur 1 zeigt eine transmissionselektronenmikroskopische (TEM) Aufnahme eines erfindungsgemäßen beschichteten Aluminiumoxidplättchens (vgl. Beispiel 2). Das Aluminiumoxidplättchen (1) weist eine sehr gleichmäßige Dicke auf und ist von einer SiO₂-Schicht (2) (Beschichtung A, hell) sowie einer Eisenoxidschicht (3) (Beschichtung B, dunkel) umhüllt.

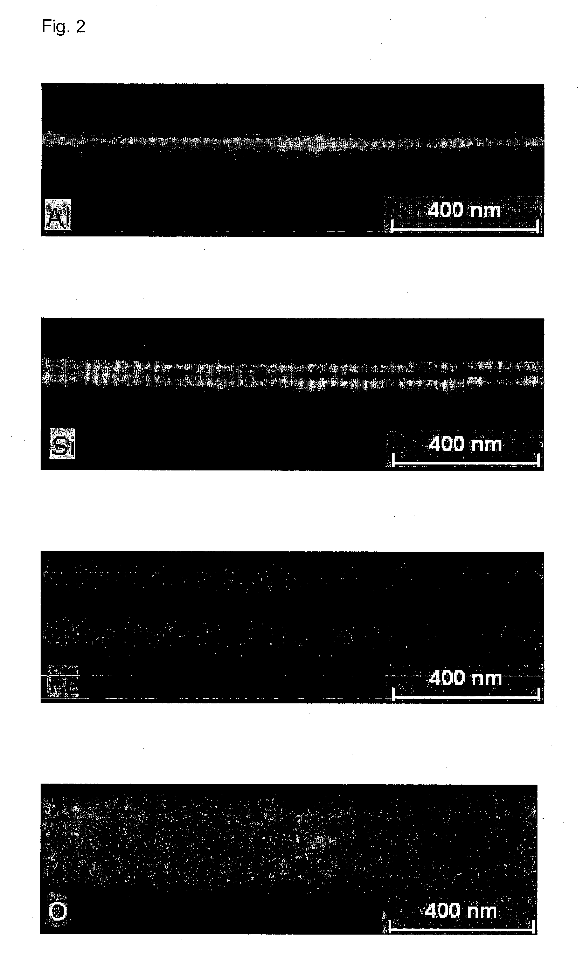

Figur 2 zeigt verschiedene TEM-Aufnahmen eines erfindungsgemäßen beschichteten Aluminiumoxidplättchens gemäß Beispiel 4, erweitert mit energiedispersiver Röntgenanalyse (*energy-dispersive X-ray analysis,* EDX, auch energiedispersive Röntgenspektroskopie, EDS, genannt) Man sieht (von oben nach unten) den scan von Aluminium, Silicium, Eisen und Sauerstoff. Deutlich ist zu erkennen, dass der Sauerstoff auch in der Aluminiumschicht vorhanden ist.

Die nachstehenden Beispiele dienen als weitere Erläuterung der vorliegenden Erfindung, ohne darauf beschränkt zu sein.

### Beispiel 1 - dünne Aluminiumplättchen mit SiO₂ (50 nm) und Eisenoxidbeschichtung (110 nm)

Zunächst wurden 10 g Al-Plättchen (Dicke zwischen 10 nm und 20 nm, d₅₀ = 15 µm) mittels Sol-Gel-Verfahren unter Verwendung von Tetraethylorthosilikat (TEOS) mit 60 g SiO₂ beschichtet. In einem Rundkolben mit Rückflußkühler und Rührer wurden diese Al-Plättchen mit 500 mL deionisiertem Wasser versetzt und unter Rühren auf 75°C erwärmt. Der pH-Wert wurde durch Hinzufügen einer 10%igen NaOH-Lösung auf einen Wert von 3,2 eingestellt. Zu dem Reaktionsgemisch wurden 700 g einer 40%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war.

Die erhaltenen beschichteten Aluminiumplättchen wurden 290 Minuten bei einer Aufheizrate von 2°C/Minute bis ca. 600° calciniert und mit einem Sieb (Maschenweite 32 µm) gesiebt. Das erhaltene Produkt wurde einer Bewertung seiner Farbeigenschaften unterzogen.

### Beispiel 2 - dünne Aluminiumplättchen mit SiO₂ (50 nm) und Eisenoxidbeschichtung (100 nm)

Zunächst wurden 10 g Al-Plättchen (Dicke zwischen 10 nm und 20 nm, d₅₀ = 15 µm) mittels Sol-Gel-Verfahren unter Verwendung von Tetraethylorthosilikat (TEOS) mit 60 g SiO₂ beschichtet. In einem Rundkolben mit Rückflußkühler und Rührer wurden diese Al-Plättchen mit 500 mL deionisiertem Wasser versetzt und unter Rühren auf 75°C erwärmt. Der pH-Wert wurde durch Hinzufügen einer 10%igen NaOH-Lösung auf einen Wert von 3,2 eingestellt. Zu dem Reaktionsgemisch wurden 650 g einer 40%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war.

Die erhaltenen beschichteten Aluminiumplättchen wurden 290 Minuten bei einer Aufheizrate von 2°C/Minute bis ca. 600° calciniert und mit einem Sieb (Maschenweite 32 µm) gesiebt. Das erhaltene Produkt wurde einer Bewertung seiner Farbeigenschaften unterzogen.

### Beispiel 3 - dünne Aluminiumplättchen mit SiO₂ (15 nm) und Eisenoxidbeschichtung (100 nm)

Zunächst wurden 10 g Al-Plättchen (Dicke zwischen 20 nm und 30 nm, d₅₀ = 20 µm) mittels Sol-Gel-Verfahren unter Verwendung von Tetraethylorthosilikat (TEOS) mit 10 g SiO₂ beschichtet. In einem Rundkolben mit Rückflußkühler und Rührer wurden diese Al-Plättchen mit 500 mL deionisiertem Wasser versetzt und unter Rühren auf 75°C erwärmt. Der pH-Wert wurde durch Hinzufügen einer 10%igen NaOH-Lösung auf einen Wert von 3,2 eingestellt. Zu dem Reaktionsgemisch wurden 400 g einer 40%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war.

Die erhaltenen beschichteten Aluminiumplättchen wurden 290 Minuten bei einer Aufheizrate von 2°C/Minute bis ca. 600° calciniert und mit einem Sieb (Maschenweite 32 µm) gesiebt. Das erhaltene Produkt wurde einer Bewertung seiner Farbeigenschaften unterzogen.

### Beispiel 4 - dünne Aluminiumplättchen mit SiO₂ (25 nm) und Eisenoxidbeschichtung (110 nm)

Zunächst wurden 10 g Al-Plättchen (Dicke zwischen 10 nm und 20 nm, dso = 15 µm) mittels Sol-Gel-Verfahren unter Verwendung von Tetraethylorthosilikat (TEOS) mit 30 g SiO₂ beschichtet. In einem Rundkolben mit Rückflußkühler und Rührer wurden diese Al-Plättchen mit 500 mL deionisiertem Wasser versetzt und unter Rühren auf 75°C erwärmt. Der pH-Wert wurde durch Hinzufügen einer 10%igen NaOH-Lösung auf einen Wert von 3,2 eingestellt. Zu dem Reaktionsgemisch wurden 750 g einer 40%igen FeCl₃-Lösung gegeben, wobei der pH-Wert durch gleichzeitige Zugabe einer 10%igen NaOH-Lösung im Wesentlichen konstant bei 3,2 gehalten wurde. Nach vollständiger Zugabe der FeCl₃-Lösung wurde das Gemisch für weitere 15 Minuten gerührt, um eine vollständige Fällung zu gewährleisten. Der pH-Wert wurde dann durch Zutropfen einer 10%igen NaOH-Lösung über eine Dauer von 30 Minuten auf einen Wert von 7,0 erhöht. Nach 30 Minuten weiteren Rührens wurde das beschichtete Pigment von der überstehenden Reaktionslösung durch Filtrieren abgetrennt und gewaschen, bis es salzfrei war.

Die erhaltenen beschichteten Aluminiumplättchen wurden 290 Minuten bei einer Aufheizrate von 2°C/Minute bis ca. 600° calciniert und mit einem Sieb (Maschenweite 32 µm) gesiebt. Das erhaltene Produkt wurde einer Bewertung seiner Farbeigenschaften unterzogen. In Fig. 3 sind die entsprechenden TEM-Aufnahmen erweitert mit EDX-Analyse dargestellt, die belegen, dass das metallische Al-Substratplättchen vollständig oxidiert ist.

### Beispiel 5 - dünne Aluminiumplättchen mit Titandioxidbeschichtung

Zunächst wurden 10 g Al-Plättchen (Dicke zwischen 14 nm und 18 nm, d₅₀ = 14 µm) mittels Sol-Gel-Verfahren unter Verwendung von Tetraethylorthosilikat (TEOS) mit 60 g SiO₂ beschichtet.

Anschließend wurden mittels TiOCl in 500 ml Wasser unter Rühren, bei pH 2,0 und 75°C 163 g Titandioxid aufgebracht. Nach der Reaktion wurde der pH-Wert auf 5,5 angehoben. Die so hergestellten, beschichteten Aluminiumflakes wurden filtriert und sorgfältig gewaschen und bei 120° getrocknet.

Anschließend wurde die beschichteten Aluminiumflakes mit 2°C/Minute auf 800°C aufgeheizt. Nach dem Abkühlen wurde das Fertigpigment noch mit einem 25 µm Sieb gesiebt und anschließend koloristisch geprüft.

### Beispiel 6 - koloristische Untersuchungen

Zur koloristischen Prüfung wurde jeweils ein Lack mit einer Pigmentkonzentration von 12 Gew.-% (im Falle von körperfarbenen Perlglanzpigmenten nach Beispiel 1 und Beispiel 4 - vgl. Tabellen 1 und 2) bzw. 6 Gew.-% (im Falle von transparenten Perlglanzpigmenten nach Beispiel 5 - vgl. Tabelle 3) bezogen auf das Bindemittel hergestellt. Mittels eines Rakels wurde eine schwarze Platte mit einer Schichtdicke von 12 µm beschichtet. Die Farbdaten wurden mit einem Byk-mac der Firma Byk Additive und Instrumente ermittelt.

Als Vergleich wurden handelsübliche Pigmente der Firma Merck (Iriodin^{®} 502 Rotbraun und Iriodin^{®} 9225 Rutile Blue Pearl SW) und der Firma BASF (Mearlin^{®} Exterior CFS Super Copper 3503Z) herangezogen, wobei es sich um Perlpigmente mit anorganischem Substrat auf Basis von Mica Glimmer handelt.

Dabei wurden Perlglanzpigmente mit jeweils fast gleichem Interferenzfarbwinkel verglichen und die Werte gegenübergestellt (vgl. Tabellen 1 bis 3). Entscheidend hierbei sind die Chromwerte der Interferenz (C), bei körperfarbenen Perlglanzpigmenten ist auch die Intensität der Körperfarbe und das Deckvermögen ausgedrückt im ΔE Wert von Bedeutung.

Tabelle 1 zeigt Resultate für Perlglanzpigmente mit Körperfarbe, wobei die erfindungsgemäßen Perlglanzpigmente aus Beispiel 1 mit Mearlin^{®} Exterior CFS Super Copper 3503Z der Firma BASF verglichen werden.

Tabelle 2 zeigt Resultate für Perlglanzpigmente mit Körperfarbe, wobei die erfindungsgemäßen Perlglanzpigmente aus Beispiel 4 mit Iriodin^{®} 502 Rotbraun der Firma Merck und Mearlin^{®} Exterior CFS Super Copper 3503Z der Firma BASF verglichen werden.

Tabelle 3 zeigt Resultate für transparente blaue Perlglanzpigmente, wobei das erfindungsgemäße Perlglanzpigment aus Beispiel 5 mit Iriodin^{®} 9225 Rutile Blue Pearl SW der Firma Merck verglichen wird.

**Tabelle 1; Orange Pigmente**

| **Nr.** | **L* 45°/15°** | **a* 45°/15°** | **b* 45°/15°** | **C 45°/15°** | **L* 45°/45°** | **a* 45°/45°** | **b* 45°/45°** | **C 45°/45°** | **ΔE 45°** |
|---|---|---|---|---|---|---|---|---|---|
| | | | | | | | | | |
| 1 | 80,8 | 80,8 | 61,4 | **81,7** | 25,1 | 27,8 | 28,5 | **39,8** | **18,2** |
| Mearlin^{®} 3503Z | 90,7 | 43,9 | 37,6 | **57,8** | 23,2 | 20,0 | 20,1 | **28,4** | **24,0** |

**Tabelle 2: Orangerote Pigmente**

| **Nr.** | **L* 45°/15°** | **a* 45°/15°** | **b* 45°/15°** | **C 45°/15°** | **L* 45°/45°** | **a* 45°/45°** | **b* 45°/45°** | **C 45°/45°** | **ΔE 45°** |
|---|---|---|---|---|---|---|---|---|---|
| | | | | | | | | | |
| | | | | | | | | | |
| 4 | 89,9 | 58,0 | 72,0 | **92,4** | 29,1 | 28,9 | 36,8 | **46,7** | **7,7** |
| Iriodin^{®} 502 | 92,0 | 92,0 | 47,9 | **66,1** | 21,8 | 20,2 | 21,0 | **29,1** | **27,0** |

**Tabelle 3**

| **Nr.** | **L* 45°/15°** | **a* 45°/15°** | **b* 45°/15°** | **C 45°/15°** |
|---|---|---|---|---|
| 5 | 58,4 | 58,4 | 13,1 | **59,9** |
| Iriodin^{®} 9225 | 55,6 | 6,7 | 45,8 | **46,3** |

Aus den Tabellen 1 bis 3 ist ersichtlich, dass die erfindungsgemäßen Perlglanzpigmente im Vergleich zu handelsüblichen Pigmenten bei vergleichbaren Interferenzfarbwinkeln deutlich höhere Chromwerte zeigen.

Insbesondere ist die Chroma der Körperfarbe (C45°/45°) des Beispiels 1 deutlich höher als die entsprechende Chroma des handelsüblichen Pigments Mearlin^{®} 3503Z. Auch die Interferenzchroma (C45°/15°) des Beispiels 1 ist im Vergleich zu Mearlin^{®} 3503Z verbessert.

Aus dem Vergleich des Beispiels 4 mit Iriodin^{®} 502 zeigt sich ebenfalls eine deutliche Verbesserung der Chroma der Körperfarbe (C45°/45°) sowie der Interferenzchroma (C45°/15°).

Darüber hinaus belegen die Werte in den Tabellen 1 und 2, dass die erfindungsgemäßen Perlglanzpigmente im Falle von körperfarbenen Pigmenten einen besonders geringen Farbabstand ΔE und damit ein besonders hohes Deckvermögen aufweisen.

Bei den transparenten Perlglanzpigmenten mit blauer Interferenz zeigt sich, dass die Chroma im Interferenzwinkel (C45°/15°) des Beispiels 5 gegenüber dem handelsüblichen Pigment deutlich überlegen ist.

## Patentansprüche

1. Verfahren zur Herstellung von Perlglanzpigmenten, umfassend die Schritte
des Bereitstellens von monolithisch aufgebauten metallischen Substratplättchen mit einer durchschnittlichen Dicke von 1 bis 40 nm und einem Formfaktor, ausgedrückt durch das Verhältnis der mittleren Größe zur durchschnittlichen Dicke, von mindestens 80,
des Beschichtens der metallischen Substratplättchen mit einer umhüllenden im Wesentlichen transparenten Beschichtung A aus mindestens einem niedrigbrechenden Metalloxid und/oder Metalloxidhydrat, das einen Brechungsindex von weniger als 1,8 aufweist,
des Beschichtens der derart beschichteten metallischen Substratplättchen mit mindestens einer Interferenzschicht in Form einer Beschichtung B aus mindestens einem hochbrechenden Metalloxid, das einen Brechungsindex von mindestens 1,8 aufweist,
wobei die Beschichtungsschritte durch hydrolytische Zersetzung einer oder mehrerer organischer Metallverbindungen und/oder durch Fällung eines oder mehrerer gelöster Metallsalze erfolgen, und
anschließend des Calcinierens der derart beschichteten Substratplättchen bei 500 bis 1200°C, wodurch das metallische Substratplättchen in das entsprechende Metalloxid überführt wird.

2. Verfahren nach Anspruch 1, wobei die metallischen Substratplättchen aus Aluminium bestehen.

3. Verfahren nach Anspruch 2, wobei vor dem Calcinierungsschritt das Stoffmengenverhältnis α von Sauerstoff in beschichteten Substratplättchen, der nicht an Aluminium gebunden ist, zu Aluminium mindestens 3 beträgt.

4. Verfahren nach einem der Ansprüche 1 bis 3, wobei die Beschichtung B im Wesentlichen aus einem hochbrechenden Metalloxid, ausgewählt aus mindestens einem von Eisen(III)oxid, Cobalt(II)oxid, Chrom(III)oxid, Nickel(II)oxid, Kupfer(I/II)oxid, Vanadium(V)oxid, Titan(III)oxid, Titandioxid, Antimon(III)oxid, Zink(II)oxid und/oder Zirkoniumdioxid, aufgebaut ist.

5. Verfahren nach einem der Ansprüche 1 bis 4, wobei die Beschichtung A aus mindestens einer Beschichtung aufgebaut ist, ausgewählt aus Silicium(di)oxid, Siliciumoxidhydrat, Aluminiumoxid, Aluminiumoxidhydrat, Ceroxid, Zinnoxid und deren Gemische.

6. Verfahren nach einem der Ansprüche 1 bis 5, wobei die metallischen Substratplättchen eine durchschnittliche Dicke von 25 nm oder weniger aufweisen.

7. Verfahren nach einem der Ansprüche 1 bis 6, wobei die metallischen Substratplättchen eine mittlere Größe d₅₀ von 5 bis 100 µm aufweisen.

8. Verfahren nach einem der Ansprüche 1 bis 7, wobei die durchschnittliche Dicke der Beschichtung A 5 bis 50 nm oder mehr als 50 nm beträgt.

9. Verfahren nach einem der Ansprüche 1 bis 8, wobei die durchschnittliche Dicke der Beschichtung B 20 bis 250 nm beträgt.

10. Verfahren nach einem der Ansprüche 1 bis 9, weiter umfassend den Schritt des Bereitstellens einer weiteren Beschichtung C auf der Beschichtung B ausgewählt aus Metalloxiden, Metalloxidhydraten und/oder organischen Silanverbindungen.

11. Perlglanzpigmente, erhältlich durch das Verfahren nach einem der Ansprüche 1 bis 10, umfassend
monolithisch aufgebaute Substratplättchen aus einem Metalloxid mit einer durchschnittlichen Dicke von 1 bis 40 nm und einem Formfaktor, ausgedrückt durch das Verhältnis der mittleren Größe zur durchschnittlichen Dicke, von mindestens 80, die von mindestens einer im Wesentlichen transparenten Beschichtung A aus mindestens einem niedrigbrechenden Metalloxid und/oder Metalloxidhydrat, das einen Brechungsindex von weniger als 1,8 aufweist, umhüllt sind, und
mindestens eine Interferenzschicht in Form einer Beschichtung B aus mindestens einem hochbrechenden Metalloxid, das einen Brechungsindex von mindestens 1,8 aufweist.

12. Perlglanzpigmente nach Anspruch 11, wobei die Substratplättchen aus Al₂O₃ bestehen.

13. Perlglanzpigmente nach Anspruch 11 oder 12, wobei die Substratplättchen eine durchschnittliche Dicke von 25 nm oder weniger aufweisen.

14. Perlglanzpigmente nach einem der Ansprüche 11 bis 13, wobei die durchschnittliche Dicke der Beschichtung A 5 bis 50 nm oder mehr als 50 nm beträgt.

15. Verwendung der Perlglanzpigmente nach einem der Ansprüche 11 bis 14 zur Einfärbung von Lacken, Druckfarben, Tinten, Kunststoffen, Gläsern, keramischen Produkten und Zubereitungen der dekorativen Kosmetik.
